# EUROPEAN PATENT APPLICATION

(11) **EP 4 102 284 A1**
(43) Date of publication of application: **14.12.2022**
(21) Application number: 21767353.2
(22) Date of filing: 03.03.2021
(51) Int. Cl.: G02B 21/06, A61B 3/135

(54) **SLIT LAMP MICROSCOPE**

(30) Priority: 10.03.2020 JP 2020041178
(71) Applicant: Sony Group Corporation, Tokyo 108-0075 (JP)
(72) Inventor: KOBAYASHI, Motoaki, Tokyo 108-0075 (JP); OOTSUKI, Tomoyuki, Tokyo 108-0075 (JP); SOMA, Yoshio, Tokyo 108-0075 (JP)
(74) Representative: D Young & Co LLP
(86) International application number: PCT/JP2021/008166
(87) International publication number: WO 2021/182230

(57) **Abstract**

A slit lamp microscope according to an embodiment of the present technology includes an illumination optical system, an imaging optical system, and a display unit. The illumination optical system emits slit light toward an eye to be examined. The imaging optical system images light reflected by the eye to be examined. The display unit has a display surface that displays a captured image obtained by imaging the eye to be examined, in which a relationship that a direction perpendicular to the display surface is parallel to a predetermined surface direction including an imaging direction of the imaging optical system is maintained. Accordingly, a novel operation can be performed.

## Description

### Technical Field

The present technology relates to a slit lamp microscope.

### Background Art

A slit lamp microscope described in Patent Literature 1 includes a background illumination unit capable of illuminating a region surrounding an emission region of slit light with background light. The background illumination unit includes a unit case capable of housing a background light source, a battery that supplies the background light source with electric power, and a control unit that controls the background light source. The unit case is removably attached to an arbitrary position of the slit lamp microscope. Accordingly, the region surrounding the emission region of the slit light is illuminated with the background light at an arbitrary angle (paragraphs [0024] and [0040], Fig. 1, and the like of Patent Literature 1).

### Citation List

### Patent Literature

Patent Literature 1: Japanese Patent Application Laid-open No. 2019-170636

### Disclosure of Invention

### Technical Problem

In an operation of a conventional slit lamp microscope as in Patent Literature 1, a user who is an observer needs to perform a diagnosis while maintaining his or her posture of looking into eyepieces, which imposes a physical burden on the user. It is thus desirable to provide a new technology in the slit lamp microscope for reduction and the like of the physical burden on the user.

In view of the above-mentioned circumstances, it is an objective of the present technology to provide a slit lamp microscope capable of performing a novel operation for the slit lamp microscope.

### Solution to Problem

In order to accomplish the above-mentioned objective, a slit lamp microscope according to an embodiment of the present technology includes an illumination optical system, an imaging optical system, and a display unit.

The illumination optical system emits slit light toward an eye to be examined.

The imaging optical system images light reflected by the eye to be examined.

The display unit has a display surface that displays a captured image obtained by imaging the eye to be examined, in which a relationship that a direction perpendicular to the display surface is parallel to a predetermined surface direction including an imaging direction of the imaging optical system is maintained.

In this slit lamp microscope, the display surface that displays the captured image obtained by imaging the eye to be examined is provided and the relationship that the direction perpendicular to the display surface is parallel to the predetermined surface direction including the imaging direction of the imaging optical system is maintained. Accordingly, a novel operation can be performed.

The captured image may be a three-dimensional image.

The display unit may move in conjunction with a movement of the imaging optical system.

The display unit may move to maintain a predetermined distance from a reference position.

The imaging optical system may be movable in an arc shape, using the eye to be examined as a basis. In this case, the display unit may move in a same direction with respect to the movement of the imaging optical system.

The display unit may be configured to tilt the display surface.

The display unit may be fixed to the imaging optical system directly or via another member and configured to move integrally with the imaging optical system.

The slit lamp microscope may further include a drive control unit that moves the display unit in conjunction with a movement of the imaging optical system.

The drive control unit may move the imaging optical system on the basis of a movement of the display unit.

The slit lamp microscope may further include an operation unit capable of operation input related to a movement of at least one of the imaging optical system or the display unit.

The drive control unit may move the display unit on the basis of a movement instruction to the imaging optical system, the movement instruction being input by the operation unit.

The drive control unit may move the imaging optical system on the basis of a movement instruction to the display unit, the movement instruction being input by the operation unit.

### Brief Description of Drawings

[Fig. 1] A diagram schematically showing the outer appearance of a slit lamp microscope.
[Fig. 2] A schematic diagram showing a relationship between an imaging direction and a direction perpendicular to a display surface.
[Fig. 3] A block diagram showing a functional configuration example of the slit lamp microscope.
[Fig. 4] A schematic diagram as the slit lamp microscope is viewed in a direction of a vertical axis.
[Fig. 5] A diagram schematically showing the outer appearance of a slit lamp microscope.
[Fig. 6] A block diagram showing a functional configuration example of the slit lamp microscope.
[Fig. 7] A flowchart showing an operation example of the slit lamp microscope.
[Fig. 8] A schematic diagram as the slit lamp microscope is viewed in the direction of the vertical axis.
[Fig. 9] A diagram schematically showing the outer appearance of a slit lamp microscope.
[Fig. 10] A schematic diagram as the slit lamp microscope is viewed in the direction of the vertical axis.
[Fig. 11] A flowchart showing another operation example of the slit lamp microscope.

### Mode(s) for Carrying Out the Invention

Hereinafter, embodiments according to the present technology will be described with reference to the drawings.

### [Configuration of Slit Lamp Microscope]

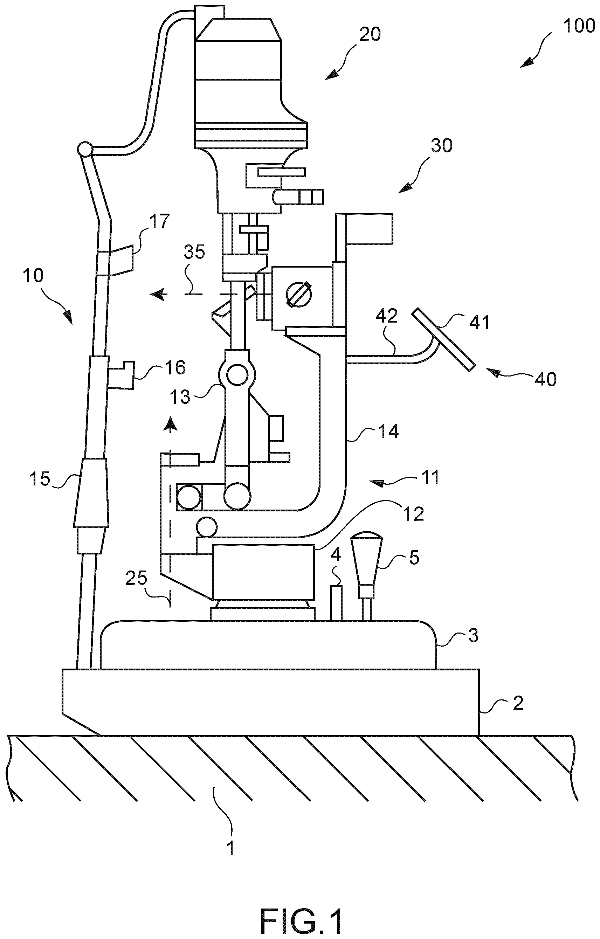

Fig. 1 is a diagram schematically showing the outer appearance of a slit lamp microscope according to the present technology.

As shown in Fig. 1, a slit lamp microscope 100 includes a placement portion 10, an illumination optical system 20, and an imaging optical system 30.

The slit lamp microscope 100 is installed in a table 1.

The placement portion 10 includes a pedestal 2 fixed on an upper surface of the table 1, a base 3 provided on an upper surface of the pedestal 2, a support 11 provided on an upper surface of the base 3, and a chin rest stand 15 supported by the pedestal 2.

The base 3 includes an illumination operation handle 4 and an imaging operation handle 5.

The illumination operation handle 4 is capable of operating an illumination support arm 13. Moreover, the illumination operation handle 4 is capable of performing operations related to the illumination optical system 20. For example, the illumination operation handle 4 is capable of operating the width of slit light, the amount of light, the wavelength, and the like.

The imaging operation handle 5 is capable of operating an imaging support arm 14. Moreover, the imaging operation handle 5 is capable of performing operations related to the imaging optical system 30. For example, the imaging operation handle 5 is capable of operating the imaging timing of an eye to be examined, the shutter open/close time, the imaging scale, and the like.

The support 11 includes a pedestal 12, the illumination support arm 13, and the imaging support arm 14.

The pedestal 12 is provided on the upper surface of the base 3 and the illumination support arm 13 and the imaging support arm 14 stand on the pedestal 12. Moreover, the illumination support arm 13 and the imaging support arm 14 are each capable of independently rotating, using a common vertical axis 25 as the center.

The illumination support arm 13 has an upper portion, to which the illumination optical system 20 is attached, and supports the illumination optical system 20. The illumination support arm 13 is rotated manually and electrically. The rotation of the illumination support arm 13 enables the illumination optical system 20 to turn around the chin rest stand 15 (the patient's eye to be examined). Moreover, the illumination support arm 13 is movable in upper and lower directions. That is, the illumination support arm 13 is capable of varying the angle of elevation and the angle of depression of slit light emitted to the eye to be examined.

The imaging support arm 14 has an upper portion, to which the imaging optical system 30 is attached, and supports the imaging optical system 30. The imaging support arm 14 is rotated manually and electrically. The rotation of the imaging support arm 14 enables the imaging optical system 30 to turn around the chin rest stand 15 (the patient's eye to be examined).

It should be noted that a configuration for rotating the illumination support arm 13 and the imaging support arm 14 is not limited. For example, a transmission mechanism such as combined gears and a rack and pinion, an actuator that generates driving force, and the like may be used.

The chin rest stand 15 is disposed in an imaging direction 35 of the imaging optical system 30. The chin rest stand 15 includes a chin rest portion 16 and a forehead rest portion 17. When the patient rests his or her head on the chin rest portion 16 and the forehead rest portion 17, the eye to be examined can be observed.

The illumination optical system 20 emits slit light toward the eye to be examined. A specific configuration of the illumination optical system 20 is not limited. For example, a filter for blocking a part of an emission region when forming slit light may be attached to the light source. In addition to this, the number of light sources, a white light generation method, the positions and the number of mirrors, the use or non-use of a wavelength plate, and the positional relationship between the respective configurations, and the like may be arbitrarily determined.

The imaging optical system 30 images light reflected by the eye to be examined. For example, the imaging optical system 30 includes a pair of left right cameras and the like. That is, the eye to be examined can be observed from two positions. It should be noted that a specific configuration of the imaging optical system 30 is not limited. For example, the imaging optical system 30 may include an optical system for changing an observation image of the eye to be examined and the scale of the captured image.

Further, an imaging device and an imaging element for imaging the eye to be examined are not limited. For example, an image sensor such as a complementary metal-oxide semiconductor (CMOS) sensor and a charge coupled device (CCD) sensor may be used.

It should be noted that reflection light may include scattered light and the like from the periphery of the eye to be examined other than slit light reflected from the eye to be examined.

It should be noted that a configuration of the slit lamp microscope 100 is not limited. For example, a configuration or operation device capable of moving the support 11 forward/rearward, leftward/rightward, and upward/downward may be provided.

In this embodiment, a display unit 40 is connected to the imaging support arm 14 via a display unit support arm 42. That is, the display unit 40 rotates following the rotation of the imaging support arm 14.

The display unit 40 displays a captured image of the eye to be examined that has been imaged by the imaging optical system 30. The captured image is a three-dimensional image. For example, a see-through organic EL display, a liquid-crystal display or liquid-crystal display element (LCD) display, or the like is used as the display unit 40.

In this embodiment, the display unit 40 has a display surface 41 that displays a captured image obtained by imaging the eye to be examined. Moreover, a relationship that a direction perpendicular to the display surface 41 is parallel to a predetermined surface direction including the imaging direction 35 of the imaging optical system 30 is maintained.

Accordingly, a user who is an observer (e.g., a doctor) who views the display unit 40 does not need anymore to look into eyepieces mounted on a conventional slit lamp microscope. That is, the user is enabled to observe the eye to be examined in a head-up environment. It should be noted that in the present disclosure, the head-up is a state not looking into the eyepieces. Moreover, a surgical operation conducted in the state not looking into the eyepieces will be referred to as head-up surgery.

It should be noted that the present technology is not limited to the three-dimensional image display. For example, autostereoscopic display may be employed, display using polarized glasses may be employed, or display using liquid-crystal shutter glasses may be employed.

It should be noted that the position and size of the display unit 40 are not limited. For example, the display unit 40 may be provided at a height depending on the user's line of sight. Moreover, a drive mechanism for changing the height may be configured.

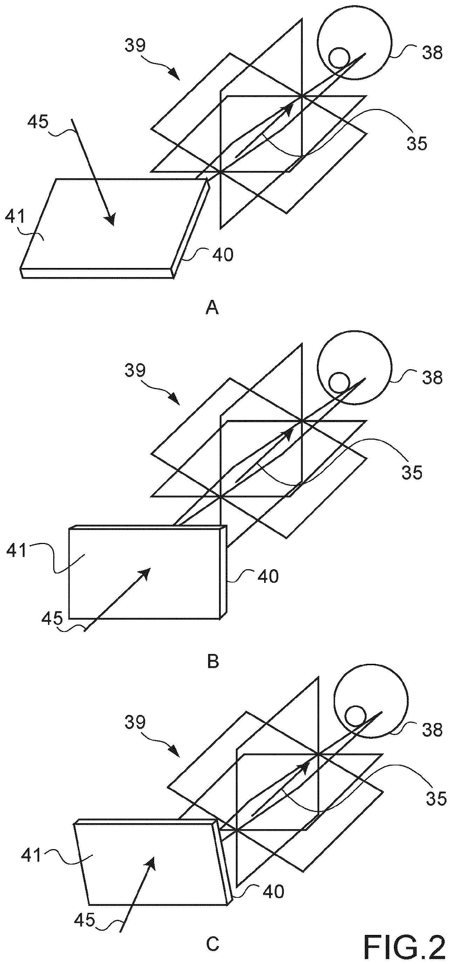

Fig. 2 is a schematic diagram showing a relationship between the imaging direction and the direction perpendicular to the display surface.

Fig. 2 shows the imaging direction 35 of the imaging optical system 30 that images an eye to be examined 38 and a predetermined surface 39 including the imaging direction 35. Moreover, the display units 40 in the respective figures are tilted in different directions and directions 45 perpendicular to the display surfaces 41 in the respective figures are different. It should be noted that in Fig. 2, the illustration of the imaging optical system 30 is omitted. Moreover, in Fig. 2, the positional relationship between the display unit 40, the eye to be examined 38, the imaging optical system 30, and the like are simplified.

In this embodiment, as shown in Fig. 2A to C, a relationship that the direction 45 perpendicular to the display surface 41 is parallel to the direction of the predetermined surface 39 including the imaging direction 35 of the imaging optical system 30 is maintained. That is, a configuration capable of varying the angle of elevation and the angle of depression of the display unit support arm 42 to cause the display surface 41 to constantly face the user so that the user can easily view the display unit 40 can be employed.

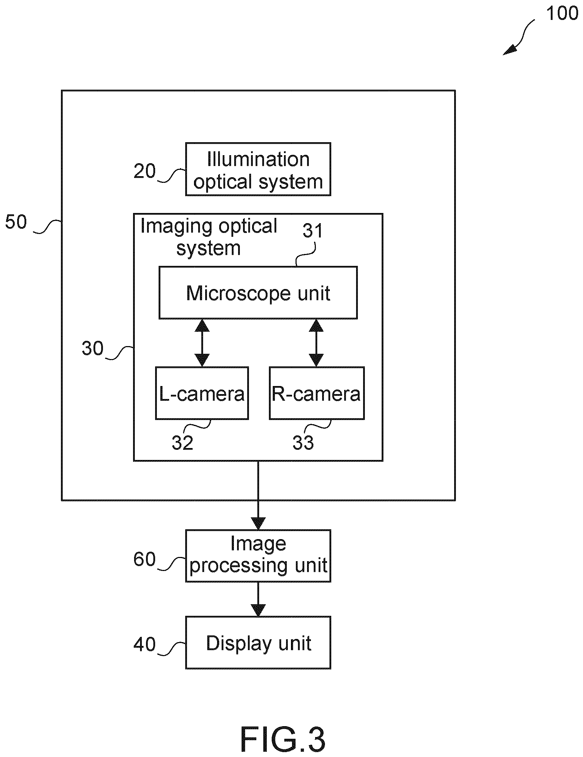

Fig. 3 is a block diagram showing a functional configuration example of the slit lamp microscope 100 shown in Fig. 1. As shown in Fig. 3, the slit lamp microscope 100 includes a camera unit 50, an image processing unit 60, and a display unit 40.

The camera unit 50 includes the illumination optical system 20 and the imaging optical system 30.

The imaging optical system 30 includes a microscope unit 31, an L-camera 32, and an R-camera 33.

The microscope unit 31 has a configuration for magnifying and observing the eye to be examined. For example, a lens and the like for magnifying the eye to be examined at various scales are used. The eye to be examined magnified by the microscope unit 31 is imaged by the L-camera 32 and the R-camera 33. The L-camera 32 and the R-camera 33 are capable of imaging the eye to be examined in different directions.

The image processing unit 60 is, for example, a camera control unit (CCU) and performs processing for the captured image captured by the imaging optical system 30. In this embodiment, three-dimensional processing is performed on the captured image of the eye to be examined that have been imaged by the L-camera 32 and the R-camera 33. That is, a three-dimensional captured image is generated on the basis of the positional relationship between the L-camera 32 and the R-camera 33.

The captured image generated by the image processing unit 60 is displayed on the display unit 40.

The camera unit 50, the image processing unit 60, and the display unit 40 may be connected to one another via wires or wirelessly so that they can communicate with one another. It may be possible to utilize wireless LAN communication such as Wi-Fi or near-field communication such as Bluetooth (registered trademark), for example, for the connection form between the respective devices.

It should be noted that in this embodiment, the illumination optical system 20 corresponds to an illumination optical system that emits slit light toward an eye to be examined.

It should be noted that in this embodiment, the imaging optical system 30 corresponds to an imaging optical system that images light reflected by the eye to be examined.

It should be noted that in this embodiment, the display unit 40 corresponds to a display unit including a display surface that displays the captured image obtained by imaging the eye to be examined, in which a relationship that the direction perpendicular to the display surface is parallel to the predetermined surface direction including the imaging direction of the imaging optical system is maintained.

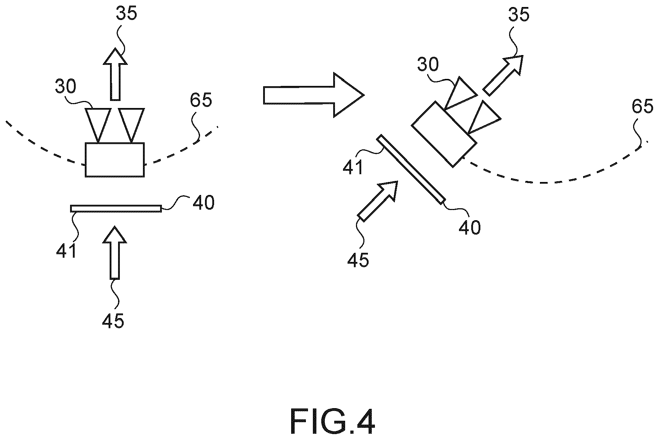

Fig. 4 is a schematic diagram as the slit lamp microscope 100 is viewed in the direction of the vertical axis 25.

As shown in Fig. 4, in this embodiment, the display unit 40 is connected to the imaging support arm 14. Therefore, due to the rotation of the imaging optical system 30, the display unit 40 and the display surface 41 on which the captured image is displayed follow the movement of the imaging optical system 30.

That is, before and after the movement, the relationship that the direction 45 perpendicular to the display surface 41 is parallel to the predetermined surface direction including the imaging direction 35 of the imaging optical system 30 is maintained. For example, when the imaging optical system 30 moves in the clockwise direction on an arc 65, the display unit 40 also moves.

### [Another Configuration of Slit Lamp Microscope]

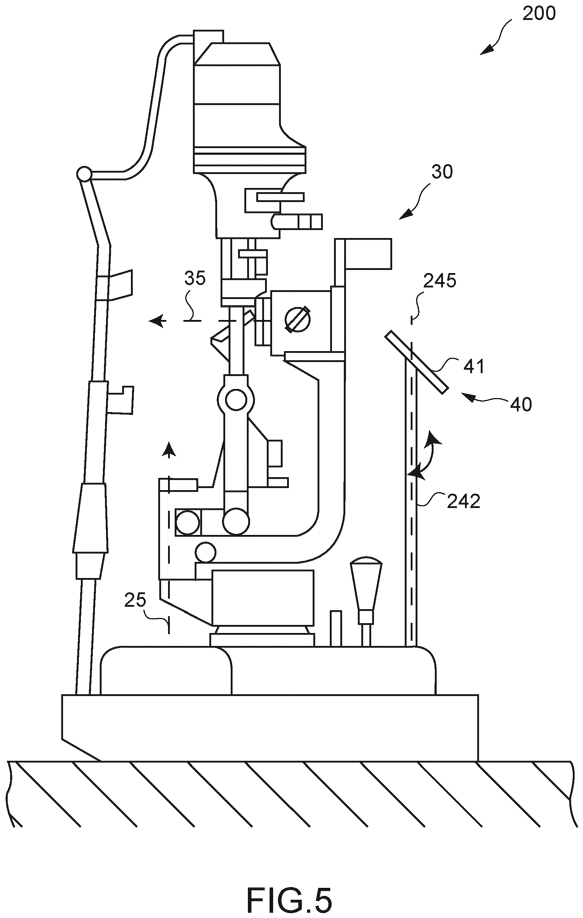

Fig. 5 is a diagram schematically showing the outer appearance of a slit lamp microscope 200. In the following descriptions, descriptions of portions similar to the configuration and actions in the slit lamp microscope 100, which have been described with Fig. 1, will be omitted or simplified.

As shown in Fig. 5, in the slit lamp microscope 200, a display unit support arm 242 that supports the display unit 40 stands on the upper surface of the base 3.

In this embodiment, the display unit support arm 242 is capable of rotating, using a rotation shaft 245 as the center. Moreover, the display unit support arm 242 rotates in conjunction with driving of the imaging support arm 14 so that a relationship that the direction perpendicular to the display surface 41 is parallel to the predetermined surface direction including the imaging direction 35 of the imaging optical system 30 is maintained.

It should be noted that in this embodiment, driving refers to physical movement of the illumination support arm 13, the imaging support arm 14, the display unit support arm, or the like in the slit lamp microscope. That is, the driving of the display unit 40 is not control of the captured image displayed on the display unit 40 but movement (change) of the position of the display unit 40 itself. Moreover, the same applies to driving of the illumination optical system 20 and driving of the imaging optical system 30.

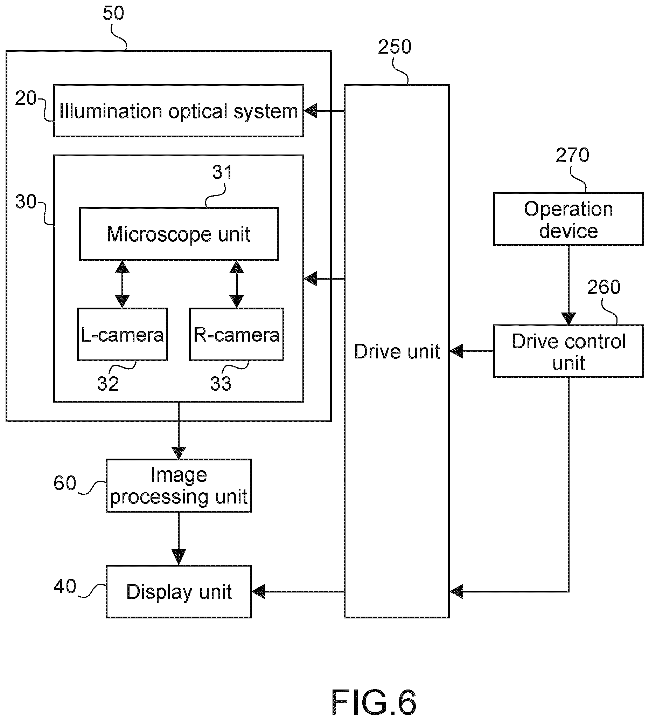

Fig. 6 is a block diagram showing a functional configuration example of the slit lamp microscope 200.

As shown in Fig. 6, the slit lamp microscope 200 includes the camera unit 50, the image processing unit 60, the display unit 40, a drive unit 250, a drive control unit 260, and an operation device 270.

The drive unit 250 drives the illumination optical system 20 and the imaging optical system 30. In this embodiment, the drive unit 250 independently drives each of the illumination support arm 13 and the imaging support arm 14 on the basis of a control command of the drive control unit 260.

Further, the drive unit 250 drives the display unit 40. In this embodiment, the display unit support arm 242 is driven in conjunction with the driving of the imaging support arm 14.

The drive control unit 260 outputs a control command to the drive unit 250 on the basis of operation input to the operation device 270. For example, on the basis of the control command to the imaging support arm 14, the control command of the display unit support arm 242 is output to the drive unit 250. Moreover, for example, on the basis of the control command to the display unit support arm 242, the control command of the imaging support arm 14 is output to the drive unit 250.

The operation device 270 is a device capable of driving the illumination optical system 20, the imaging optical system 30, and the display unit 40. For example, the operation device 270 includes the imaging operation handle 5 and the illumination operation handle 4.

It should be noted that in this embodiment, the drive control unit 260 corresponds to a drive control unit that moves the display unit in conjunction with the movement of the imaging optical system.

It should be noted that in this embodiment, the operation device 270 corresponds to an operation unit capable of operation input related to a movement of at least one of the imaging optical system or the display unit.

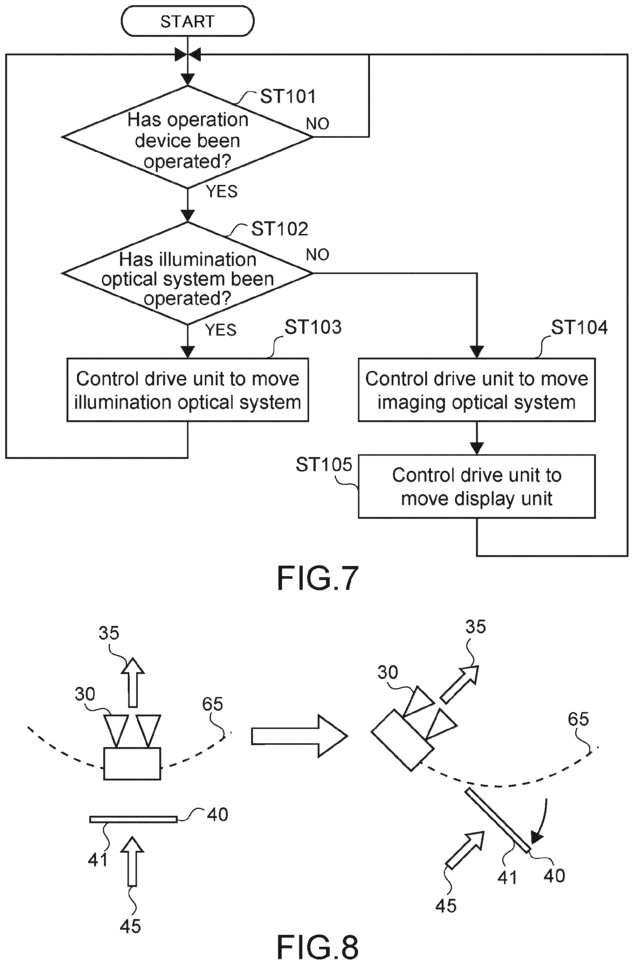

Fig. 7 is a flowchart showing an operation example of the slit lamp microscope 200.

In a case where the operation device 270 has been operated (YES in Step 101), which one, the illumination optical system 20 or the imaging optical system 30, has been operated is determined (Step 102). It should be noted that operating the illumination optical system 20 includes at least one of operating the illumination operation handle 4 or manually operating the illumination support arm 13. Operating the imaging optical system 30 includes at least one of operating the imaging operation handle 5 or manually operating the imaging support arm 14.

In a case where the illumination optical system 20 has been operated (YES in Step 102), the drive unit 250 is controlled and the illumination optical system 20 is moved (Step 103).

In a case where the illumination optical system 20 has not been operated (NO in Step 102), i.e., in a case where the imaging optical system 30 has been operated, the drive unit 250 is controlled, and the imaging optical system 30 is moved (Step 104).

The imaging optical system 30 is moved, and a control command based on the driving of the imaging optical system 30 is output to the drive unit 250. Accordingly, the display unit 40 is moved (Step 105).

Fig. 8 is a schematic diagram as the slit lamp microscope 200 is viewed in the direction of the vertical axis 25.

In this embodiment, in the display unit 40, the relationship that the direction 45 perpendicular to the display surface 41 is parallel to the predetermined surface direction including the imaging direction 35 of the imaging optical system 30 is maintained.

In Fig. 8, the imaging optical system 30 is capable of moving the arc 65, using the eye to be examined as the center. For example, it is assumed that the imaging optical system 30 has moved in the clockwise direction. In this case, the display surface 41 tilts so that the imaging direction 35 is parallel to the direction 45 perpendicular to the display surface 41.

Specifically, on the basis of a control command output to the imaging support arm 14, such a control command that the imaging direction 35 is parallel to the direction 45 perpendicular to the display surface 41 is output to the display unit support arm 242.

### [Another Configuration of Slit Lamp Microscope]

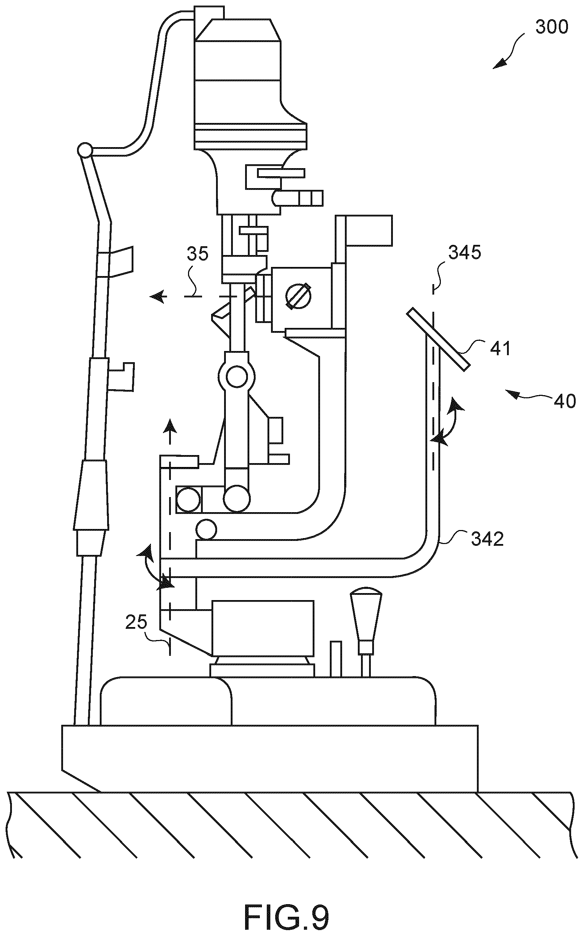

Fig. 9 is a diagram schematically showing the outer appearance of a slit lamp microscope 300.

In Fig. 9, a display unit support arm 342 that supports the display unit 40 is capable of rotating about the vertical axis 25 common to the illumination support arm 13 and the imaging support arm 14. Moreover, the display unit support arm 342 is capable of rotating about a rotation axis 345.

In this embodiment, the display unit 40 moves to maintain a predetermined distance from a reference position. The display unit support arm 342 is driven to maintain a constant distance from the user (reference position) who views the display surface 41, for example. It should be noted that a configuration for driving the display unit support arm 342 is not limited thereto. For example, a mechanism that moves the display unit support arm 342 forward/rearward so that the display unit support arm 342 is movable on the arc, using the user as the center, may be provided.

Further, in this embodiment, the display unit support arm 342 rotates in conjunction with the driving of the imaging support arm 14 so that the relationship that the direction perpendicular to the display surface 41 is parallel to the predetermined surface direction including the imaging direction 35 of the imaging optical system 30 is maintained.

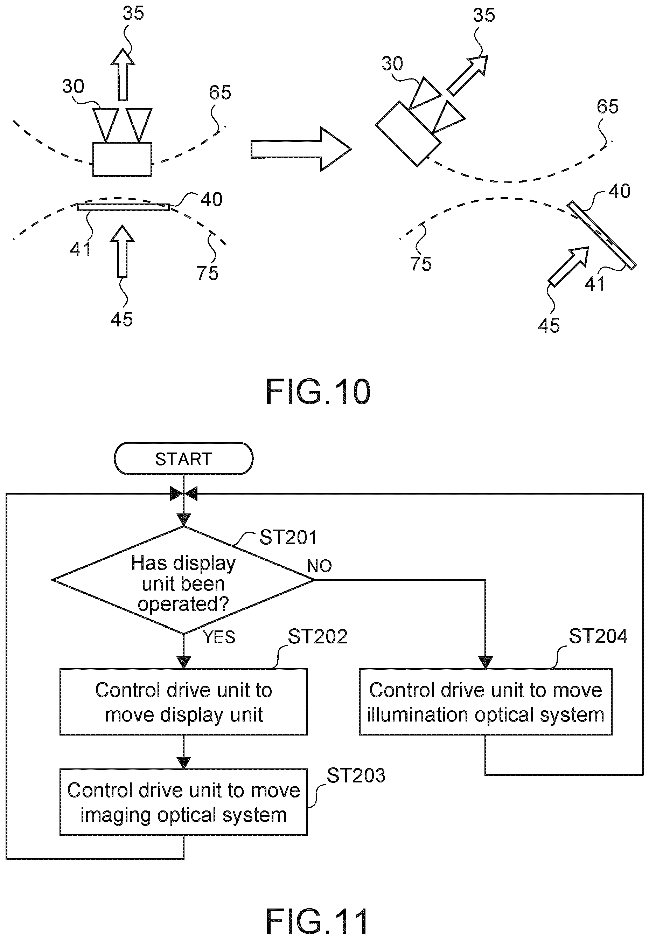

Fig. 10 is a schematic diagram as the slit lamp microscope 300 is viewed in the direction of the vertical axis 25.

In Fig. 10, the imaging optical system 30 is movable on the arc 65, using the eye to be examined as the center. For example, it is assumed that the imaging optical system 30 has moved in the clockwise direction. In this case, the imaging optical system 30 moves on an arc 75, using the user as the center, and the display surface 41 tilts to face the user side so that the imaging direction 35 is parallel to the direction 45 perpendicular to the display surface 41. That is, without changing the distance of the display unit 40 to a user who is an observer of a captured image, the display surface 41 can move to directly face the user.

Hereinabove, the slit lamp microscope 100 according to this embodiment includes the display surface 41 that displays the captured image obtained by imaging the eye to be examined and the relationship that the direction 45 perpendicular to the display surface 41 is parallel to the direction of the predetermined surface 39 including the imaging direction 35 of the imaging optical system 30 is maintained. Accordingly, a novel operation for the slit lamp microscope can be performed.

In the conventional slit lamp microscope, it has been necessary to perform a diagnosis while maintaining the posture of looking into the eyepieces. The user who uses the slit lamp microscope needs to swing the microscope portion while looking into the eyepieces and cannot avoid having a physical burden such as pain in the cervical vertebrae or waist. Therefore, it is sufficiently conceivable that the user wants to omit the swing and terminate the diagnosis.

Further, an apparatus in which the user causes the optical path to branch in the form of looking into the conventional eyepieces to thereby obtain an electronic video having a low resolution for recording does not have a structure including a display device in the casing of the original microscope. Therefore, a typical monitor device is used as the display device and is separately installed at a position spaced away from the apparatus.

In this case, another installation space is necessary in addition to the installation space for the microscope and a cable for wiring is obstructive. Moreover, in a case where the display device is a stereoscopic device, a viewing distance to the display device is increased, and therefore a stereoscopic effect is emphasized and an accurate sense of depth cannot be perceived during observation.

In view of this, in the present technology, the relationship that the direction perpendicular to the display surface on which the captured image is displayed is parallel to the predetermined surface direction including the imaging direction of the imaging optical system is maintained. Accordingly, the user does not need to move in accordance with the swing (movement) of the imaging optical system. Moreover, the user also does not need to look into the eyepieces, and therefore the user does not need anymore to take the posture that causes a heavy physical burden. As a result, the user can actively swing the imaging optical system in the diagnosis and perform a precise diagnosis.

Further, since it is possible to perform observation without looking into the eyepieces, the physical burden on the user can be reduced. That is, the user's active lifetime can be prolonged. Moreover, since the display unit moves in conjunction with the movement of the imaging optical system, it is possible to reduce the user's body movements such as changing the face direction.

Since the display unit is integral with the slit lamp microscope, it is unnecessary to additionally prepare an installation space for the display unit, and the display unit can be installed in the same installation space as the conventional slit lamp microscope. That is, replacement of the conventional slit lamp microscope can be easily performed.

Since the distance between the display surface and the user is kept optimal, observation with an accurate sense of depth in the three-dimensional display is possible. Moreover, eyestrain caused by observation of three-dimensional images in long-time use can be reduced.

Since the display unit is driven in conjunction with the driving of the imaging optical system, the operability is the same as the conventional slit lamp microscope, which prevents discomfort during the use.

### <Other Embodiments>

The present technology is not limited to the above-mentioned embodiments, and various other embodiments can be realized.

In the above-mentioned embodiments, the display unit support arm 242 or 342 is driven in conjunction with the driving of the imaging support arm 14. The present technology is not limited thereto, and the imaging support arm 14 may be driven in conjunction with the driving of the display unit support arm 242 or 342.

Fig. 11 is a flowchart showing another operation example of the slit lamp microscope 200 or 300.

In a case where the display unit 40 has been operated (YES in Step 201), the drive unit 250 is controlled, and the display unit 40 is moved (Step 202). It should be noted that operating the display unit 40 includes operating the operation device capable of operating the display unit support arm 242 or 342 and manually operating the display unit support arm 242 or 342.

The display unit 40 is moved, and the control command based on driving of the display unit 40 is output to the drive unit 250. Accordingly, the imaging optical system 30 is moved (Step 203).

In a case where the display unit 40 has not been operated (NO in Step 201), i.e., in a case where the illumination optical system 20 has been operated, the drive unit 250 is controlled, and the illumination optical system 20 is moved (Step 204).

Accordingly, since the imaging optical system 30 moves in conjunction with the movement of the display unit 40 for example electrically, it is possible to reduce the user's body movements such as changing the direction of the face as compared to Figs. 1 and 5. Moreover, the relationship that the direction in which the user observes the display surface 41 (the direction 45 perpendicular to the display surface 41) is parallel to the predetermined surface direction including the imaging direction 35 is maintained, it becomes easy to recognize the current imaging direction 35 of the imaging optical system 30.

In the above-mentioned embodiments, the three-dimensional image is used as the captured image. The present technology is not limited thereto, and the captured image may be a two-dimensional image. Accordingly, the image processing unit 60 can be omitted, and the costs can be reduced.

In the above-mentioned embodiments, the imaging optical system 30 and the display unit 40 are two-dimensionally driven using the eye to be examined as the center. The present technology is not limited thereto, and the imaging optical system 30 and the display unit 40 may have a degree of freedom in the angle of elevation and the angle of depression.

The respective configurations of the slit lamp microscopes and the display unit, the operation examples of the respective support arms, and the like that have been described with reference to the respective drawings are merely embodiments, and can be arbitrarily modified without departing from the gist of the present technology. That is, any other configurations, manufacturing flows, and the like for carrying out the present technology may be employed.

It should be noted that in the present disclosure, it is assumed that the concepts that define the shape, the size, the position relationship, the state, and the like such as "center", "middle", "uniform", "equal", "the same", "orthogonal", "parallel", "symmetric", "extending", "axial", "columnar", "cylindrical", "ring-shaped", and "annular" are concepts including "substantially center", "substantially middle", "substantially uniform", "substantially equal", "substantially the same", "substantially orthogonal", "substantially parallel", "substantially symmetric", "substantially extending", "substantially axial", "substantially columnar", "substantially cylindrical", "substantially ring-shaped", "substantially annular", and the like.

For example, states included in a predetermined range (e.g., ±10% range) using "completely center", "completely middle", "completely uniform", "completely equal", "completely the same", "completely orthogonal", "completely parallel", "completely symmetric", "completely extending", "completely axial", "completely columnar", "completely cylindrical", "completely ring-shaped", "completely annular", and the like as the basis are also included.

Therefore, also in a case where the term "approximately" is not added, they can include concepts expressed by adding so-called "approximately". In contrast, states expressed with "approximately" should not be understood to exclude complete states.

At least two features of the features according to the present technology, which have been described above, may be combined. That is, the various features described in the respective embodiments may be arbitrarily combined across the respective embodiments. Moreover, the above-mentioned various effects are merely exemplary and not limitative, and other effects may be provided.

It should be noted that the present technology can also take the following configurations.
(1) A slit lamp microscope, including:
   an illumination optical system that emits slit light toward an eye to be examined;
   an imaging optical system that images light reflected by the eye to be examined; and
   a display unit having a display surface that displays a captured image obtained by imaging the eye to be examined, in which a relationship that a direction perpendicular to the display surface is parallel to a predetermined surface direction including an imaging direction of the imaging optical system is maintained.
(2) The slit lamp microscope according to (1), in which the captured image is a three-dimensional image.
(3) The slit lamp microscope according to (1) or (2), in which
   the display unit moves in conjunction with a movement of the imaging optical system.
(4) The slit lamp microscope according to (3), in which
   the display unit moves to maintain a predetermined distance from a reference position.
(5) The slit lamp microscope according to (4), in which
   the imaging optical system is movable in an arc shape, using the eye to be examined as a basis, and
   the display unit moves in a same direction with respect to the movement of the imaging optical system.
(6) The slit lamp microscope according to any one of (3) to (5), in which
   the display unit is configured to tilt the display surface.
(7) The slit lamp microscope according to any one of (3) to (6), in which
   the display unit is fixed to the imaging optical system directly or via another member and configured to move integrally with the imaging optical system.
(8) The slit lamp microscope according to any one of (3) to (7), further including
   a drive control unit that moves the display unit in conjunction with a movement of the imaging optical system.
(9) The slit lamp microscope according to (8), in which
   the drive control unit moves the imaging optical system on the basis of a movement of the display unit.
(10) The slit lamp microscope according to (8) or (9), further including
   an operation unit capable of operation input related to a movement of at least one of the imaging optical system or the display unit.
(11) The slit lamp microscope according to (10), in which
   the drive control unit moves the display unit on the basis of a movement instruction to the imaging optical system, the movement instruction being input by the operation unit.
(12) The slit lamp microscope according to (10), in which
   the drive control unit moves the imaging optical system on the basis of a movement instruction to the display unit, the movement instruction being input by the operation unit.

### Reference Signs List

- 20: illumination optical system
- 30: imaging optical system
- 35: imaging direction
- 38: eye to be examined
- 39: predetermined surface
- 40: display unit
- 41: display surface
- 45: perpendicular direction
- 100, 200, 300: slit lamp microscope

## Claims

1. A slit lamp microscope, comprising:
an illumination optical system that emits slit light toward an eye to be examined;
an imaging optical system that images light reflected by the eye to be examined; and
a display unit having a display surface that displays a captured image obtained by imaging the eye to be examined, in which a relationship that a direction perpendicular to the display surface is parallel to a predetermined surface direction including an imaging direction of the imaging optical system is maintained.

2. The slit lamp microscope according to claim 1, wherein
the captured image is a three-dimensional image.

3. The slit lamp microscope according to claim 1, wherein
the display unit moves in conjunction with a movement of the imaging optical system.

4. The slit lamp microscope according to claim 3, wherein
the display unit moves to maintain a predetermined distance from a reference position.

5. The slit lamp microscope according to claim 4, wherein
the imaging optical system is movable in an arc shape, using the eye to be examined as a basis, and
the display unit moves in a same direction with respect to the movement of the imaging optical system.

6. The slit lamp microscope according to claim 3, wherein
the display unit is configured to tilt the display surface.

7. The slit lamp microscope according to claim 3, wherein
the display unit is fixed to the imaging optical system directly or via another member and configured to move integrally with the imaging optical system.

8. The slit lamp microscope according to claim 1, further comprising
a drive control unit that moves the display unit in conjunction with a movement of the imaging optical system.

9. The slit lamp microscope according to claim 8, wherein
the drive control unit moves the imaging optical system on a basis of a movement of the display unit.

10. The slit lamp microscope according to claim 8, further comprising
an operation unit capable of operation input related to a movement of at least one of the imaging optical system or the display unit.

11. The slit lamp microscope according to claim 10, wherein
the drive control unit moves the display unit on a basis of a movement instruction to the imaging optical system, the movement instruction being input by the operation unit.

12. The slit lamp microscope according to claim 10, wherein
the drive control unit moves the imaging optical system on a basis of a movement instruction to the display unit, the movement instruction being input by the operation unit.
